(19) Europäisches Patentamt
European Patent Office
Office européen des brevets

(11) Veröffentlichungsnummer: **0 003 786**
**B1**

(12) **EUROPÄISCHE PATENTSCHRIFT**

(21) Anmeldenummer: **79100391.6**

(22) Anmeldetag: **12.02.79**

(51) Int. Cl.³: **C 07 B 19/02,**
C 07 C 101/06

# (54) Stereoselektive Spaltung von Phenylglycinderivaten und 4-Hydroxyphenylglycinderivaten mit Enzymharzen

(30) Priorität: **21.02.78 DE 2807286**

(43) Veröffentlichungstag der Anmeldung:
**05.09.79 Patentblatt 79/18**

(45) Bekanntmachung des Hinweises auf die Patenterteilung:
**10.12.80 Patentblatt 80/25**

(84) Benannte Vertragsstaaten:
**BE CH DE FR GB IT NL SE**

(56) Entgegenhaltungen:
**GB - A - 1 369 462**
**US - A - 3 816 254**
**US - A - 3 963 573**
**CHEMICAL ABSTRACT, Band 76, Nr. 15**
**10 April 1972**
**Columbus, Ohio, USA**
**T. N. PATTABIRAMAN et al**
**"Comparative studies of the specificities of α-chymotrypsin and subtilisin BPN'. Flexible subtrates"**
**Abstract Nr. 82748 f**

(73) Patentinhaber: **BAYER AG**
**Zentralbereich Patente, Marken und Lizenzen**
**D - 5090 Leverkusen 1**
**Bayerwerk (DE)**

(72) Erfinder: **Schutt, Hermann, Dr**
**Roeberstrasse 8**
**D - 5600 Wuppertal 1 (DE)**

**EP 0 003 786 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Stereoselektive Spaltung von Phenylglycinderivaten und 4-Hydroxyphenylglycinderivaten mit Enzymharzen

Die Erfindung betrifft ein neues enzymatisches Verfahren zur stereoselektiven Spaltung von DL-Phenylglycinderivaten durch Hydrolyse der Ester gruppe an N-Acyl-L-phenylglycinestern in N-Acyl-DL-phenylglycinestern durch Einwirkung von Enzymen, Trennung der N-Acyl-DL-phenylglycinester von den N-Acyl-L-phenylglycinen und gegebenenfalls anschließende saure Hydrolyse der Ester gruppen de D-Enantiomeren sowie der Acylgruppen, dadurch gekennzeichnet, daß man auf die N-Acyl-DL-phenylglycinester in Lösung trägergebundene Enzyme einwirken läßt.

D-Phenylglycin und D-4-Hydroxyphenylglycin dienen als Ausgangsstoffe für die Herstellung halbsynthetischer Antibiotika der Penicillin-Reihe. L-Phenylglycin ist Ausgangsstoff für den als Süßstoff verwendeten L-Asparagin-L-phenylglycinmethylester.

Die industriell eingeführte Methode zur Racemat-Spaltung von DL-Phenylglycin und DL-4-Hydroxyphenylglycin ist die fraktionierte Kristallisation der Salze beider Aminosäuren mit DL-Campfersulfonsäure (J. P. Greenstein und M. Winitz, Chemistry of Amino acids, Vol. 1 (1961) 658). Wegen des hohen Preises der Campfersulfonsäure muß diese möglichst vollständig wiedergewonnen werden, was technisch kaum zu realisieren ist. Es hat daher nicht an Versuchen gefehlt, Racemat-Spaltungen von Derivaten des Phenylglycins und des 4-Hydroxyphenylglycins auf völlig anderer Basis als der fraktionierten Kristallisation diastereoisomerer Verbindungen durchzuführen. Die neueren Verfahren versuchen die hohe Stereospezifität bestimmter Enzyme auszunutzen. Bei den bisher bekanntgewordenen Verfahren müssen jedoch eine Reihe von Nachteilen in Kauf genommen werden.

In der DE—OS 25 26 594 wird L-Phenylglycinamid in DL-Phenylglycinamid durch eine dazu geeignete Aminopeptidase zum L-Phenylglycin hydrolysiert und vom unveränderten D-Phenylglycinamid getrennt, wobie das verwendete Enzym Leucinaminopeptidase (EC 3.4.1.1.) teilweise an einen Träger gebunden ist. Nach diesem Verfahren kann nur in hoher Verdünnung gearbeitet werden, weil das Hydrolyseprodukt L-Phenylglycin wegen seiner Schwerlöslichkeit auf dem Enzym kristallisieren und so dessen Aktivitäten in kürzester Zeit zum Erliegen bringen würde. Die Folge ist, daß große Volumina entstehen, die zur Isolierung des gewünschten Produktes energieaufwendig konzentriert werden müssen. Entsprechendes gilt für die Verfahren der DE—OS 26 21 076 und GB—PS 1 369 462, in denen aus Phenyl- bzw. 4-Hydroxyphenylhydantoinen oder aus N-Penicillin-ReiheL-Phenylglycin ist Ausgangs-Phenylglycin und L-4-Hydroxyphenylglycin entstehen.

Es ist weiterhin aus Biochem. J. (1972), *126*, 645—657 bekannt, DL-2-Acetamido-2-phenylessigsäuremethylester mit trägerfreiem α-Chymotrypsin zu D-2-Acetamido-2-phenylessigsäuremethylester und L-2-Acetamido-2-phenylessigsäure zu spalten, wobei von den Produkten nur 60 bzw. 52,5% der Theorie erhalten werden.

Es wurde nun überraschend gefunden, daß die enzymatische Spaltung von Phenylglycinderivaten in wesentlich höherer Konzentration durchgeführt werden kann, wenn man als Ausgangsmaterial N-Acyl-DL-phenylglycinester in Lösung der enzymatischen Hydrolyse an trägergebundenen proteolytischen Enzymen unterwirft.

Aus der US—PS 3 816 254 ist bekannt, bestimmte acylierte Aminosäuren in Form ihrer racemischen Gemische mit Enzymen zur Herstellung freier L-Aminosäuren. Die eingesetzten Enzyme sind N-Acylasen und haben eine völlig andere Substratspezifität als die erfindungsgemäß verwendeten Enzyme.

Auch die nach der GB—PS 1 369 462 zur Trennung von Racematen acylierter Aminosäuren eingesetzten Enzyme sind N-Acylasen. Demgegenüber wird erfindungsgemäß die Glycin-ester-Bindung gespalten durch Einsatz von O-Acylasen.

Gemäß der auf Seite 10, Zeilen 13—15 genannten Veröffentlichungen konnte nicht erwartete werden, dab diese Enzyme im erfindungsgemäßen Verfahren erfolgreich eingesetzt werden können.

Die zu spaltenden Verbindungen entsprechen insbesondere der Formel (I)

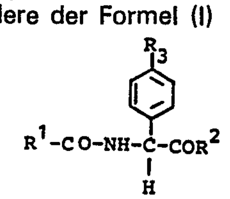

(1)

DL-Form

worin

$R^1$ Wasserstoff, den Rest einer gegebenenfalls substituierten aliphatischen oder araliphatischen Mono- oder Dicarbonsäure oder einer natürlichen oder synthetischen α-Aminocarbonsäure,

$R^2$ Alkoxy und

$R^3$ Wasserstoff, Hydroxy, Alkoxy, Aralkoxy, Aryloxy, Cycloalkloxy oder Acyloxy bedeuten.

Gegebenenfalls substituierte aliphatische Reste $R_1$ sind vor allem gegebenenfalls durch Halogen, insbesondere Fluor oder Chlor substituierte $C_1$-$C_9$-Alkyl- bzw. Alkenyl-gruppen, vorzugsweise gegebenenfalls ein-bis dreimal durch Fluor oder Chlor substituierte $C_1$-$C_4$-Alkyl bzw. Alkenylgruppen; araliphatische Reste $R_1$ sind insbesondere Phenyl-$C_1$-$C_2$-alkylgruppen, vorzugsweise der Benzylrest; die Reste $R_1$, die sich von α-Aminosäure ableiten, sind insbesondere

die von natürlichen $\alpha$-Aminosäuren, Alkoxygruppen $R_2$ sind insbesondere $C_1$-$C_4$-Alkoxy, vorzugsweise Methoxy und Äthoxy. Alkoxy $R_3$ ist insbesondere $C_1$-$C_4$-Alkoxy; Aralkoxy $R_3$ ist insbesondere Benzyloxy; Aryloxy $R_3$ ist insbesondere Phenyloxy; Cycloalkoxy $R_3$ ist insbesondere $C_5$ und $C_6$-Cycloalkoxy; Acyloxy $R_3$ ist insbesondere gegebenfalls durch 1 bis 3 Fluor oder Chlor substituiertes $C_1$-$C_4$-Alkylcarbonyloxy.

Bevorzugte Verbindungen der Formel I sind einmal solche, in denen $R_1$ Wasserstoff, Mono- Di- und Trichlormethyl, Trifluormethyl oder $\alpha$-Carboxy-$C_2$-$C_6$-alkyl, $R_2$ Methoxy oder Äthoxy und $R_3$ Wasserstoff bedeuten und zum anderen solche, in denen $R_1$ Wasserstoff, Methyl, Mono-, Di- und Trichlormethyl, Trifluormethyl oder $\omega$-Carboxy-$C_2$-$C_6$-alkyl, $R_2$ Methoxy oder Äthoxy und $R_3$ Hydroxy, Methoxy, Äthoxy oder Acetoxy bedeuten.

Als Lösungsmittel für die N-Acyl-DL-phenylglycinester und -amide kommen wasserfreie oder wasserhaltige organische Lösungsmittel, insbesondere wassermischbare Lösungsmittel in Frage, z.B. Dioxan, Äthanol und Acetonitril.

Als Enzyme kommen insbesondere proteolytische Enzyme in Frage, insbesondere Serin- und Sulfhydryl-Proteasen, vorzugsweise Subtilisin (EC 3.4.4.16.), $\alpha$-Chymo-trypsin (EC 3.4.4.5.), Papain (EC 3.4.4.10.), Ficin oder Bromelain, wobei die ersten beiden einen Serinrest im aktiven Zentrum der Aminosäurekette haben, während letztere in der Aminosäurekette Cystein als aktives Zentrum aufweisen. Subtilisin ist bevorzugt.

Pro Mol zu spaltender Verbindung werden etwa 100 mg bis 1 g an trägergebundenem Enzym eingesetzt, das wenigstens zwanzigmal wiederverwendet werden kann, während nach dem Verfahren aus Biochem. J. wesentlich größere Mengen an Enzym, das als gelöstes Produkt nur mühsam und in geringen Anteilen wiedergewonnen werden kann, gebraucht werden.

Besonders geeignet sind aus Bacillus subtilis und Bacillus licheniformis isolierte proteolytische Enzyme, die den Waschmitteln zur Entfernung von Proteinresten zugesetzt werden. Diese technischen Enzyme sind vornehmlich unter den Firmennamen Maxatase (Hersteller: Gist-Brocades N. V., Delft/Niederlande), Optimase (Hersteller: Miles-Kali-Chemie, Hannover) und Alcalase (Hersteller: Novo Industrie As, Kopenhagen/Dänemark) bekannt.

Die Eigenschaften der proteolytischen Enzyme, insbesondere ihre biochemischen Wirkungen, sind in folgenden Literaturstellen beschrieben: G. E. Perlmann und L. Lorand, Methods in Enzymology, *19* (1970) 199 bis 215; P. Desnuelle, The Enzymes, *4* (1960) 93 und G. E. Perlmann und L. Lorand, Methods in Enzymology, *19* (1970) 226 bis 244.

Die proteolytischen Enzyme können durch eine covalente Bindung über einen zur Katalyse nicht essentiellen Lysinrest an den polymeren Träger gekuppelt werden. Eine weitere Möglichkeit ist, die Adsorption des Enzyms an die Poren eines geladenen Trägers sowie die anschließende Quervernetzung mit Glutardialdehyd.

Als Enzymträger kommen Polymere, poröse Träger wie Cellulose, Dextran, Stärke, Polyacrylamidgele, Sepharose, Sephadex oder verschiedene organische Copolymerisate aus Acylamid, Methacrylaten oder Methacrylamid und Maleinsäureanhydrid nach den DE—OS 22 15 539 und 22 15 687 in Frage. Als Enzymträger können ferner Membranen aus Celluloseestern, Celluloseacetat oder Polyamiden verwendet werden, die von der Substratlösung kontinuierlich durchströmt werden (D. J. Imnan und W. E. Hornby, Biochem. J., *129* (1972) 255 bis 262).

Als besonders günstig für die Adsorption der proteolytischen Enzyme haben sich polymere Acrylsäureester wie Amberlite XAD—7 und XAD—8 sowie der schwach saure Kationenaustauscher IRC 50 (Rohm und Haas, USA) erwiesen.

Nach Quervernetzung der adsorbierten Enzyme mit 0,5—2,5% Glutadialdehyd kann ein sehr aktives und langlebiges Enzymharz hergestellt werden.

Die Kupplung der Enzyme an die porösen, unlöslichen Träger erfolgt nach an sich bekannten Methoden. Das Enzym wird zur Kupplung mit dem polymeren Träger unter optimalen Bedingungen für die Stabilität des Enzyms zur Reaktion gebracht. Die Effektivität der Kupplung kann durch Messung der enzymatischen Aktivität am Polymeren und im Waschwasser bestimmt werden. Das polymere Enzym kann bei Verwendung im Batch-Verfahren leicht durch Sedimentation oder Filtration von der Reaktionslösung abgetrennt und mehrfach eingesetzt werden. Der Enzymträger kann auch in Säulen gefüllt und in dieser Form von Substratlösung durchströmt werden.

Die in dem erfindungsgemäßen Verfahren eingesetzten N-Acylester und -amide der Phenylglycine werden durch Acylierung der entsprechenden Aminosäureester- bzw. -amidhydrochloride mit stöchiometrischen Mengen Säureanhydrid wie Acetanhydrid und anschließende Abtrennung des N-Acylderivates aus der wäßrigen Phase mit organischen Lösungsmitteln wie Chloro-form oder Methylenchlorid gewonnen.

Technische Verfahren mit trägergebundenen Enzymen sind besonders wirtschaftlich, wenn mit hohen Substratkonzentrationen gearbeitet werden kann. In hoher Konzentration liegen die N-Acylester und -amide der Phenylglycine in wäßrigem Dioxan, Äthanol oder Acetonitril als klare Lösungen vor. Von N-Acetyl-DL-phenylglycinmethylester kann in einem Dioxan-Wasser-Gemisch (1:3, volumetrisch) eine 10 %ige Lösung erhalten werden. Durch Erhöhung des Anteils an organischem Lösungsmittel kann mehr N-Acetylester gelöst werden. Die genannten Lösungsmittel wirken in hohen

Konzentrationen als Fällungsmittel für Proteine. Dieser Nachteil tritt beim erfindungsgemäßen Einsatz trägerbundener Enzyme nicht auf, da bei diesen eine Wechselwirkung der Enzymmoleküle untereinander durch die räumliche Fixierung stark behindert ist, so daß die zur Ausfällung führende Aggregation der Enzymmoleküle unterbleibt (K. Tamizawa und M. L. Bender, J. Biol. Chem., *249* (1974) 2130 bis 2134).

Die enzymatische Spaltung der N-Acyl-DL-phenylglycinester erfolgt vorzugsweise bei einer Temperatur von 20 bis 40°C in einem pH-Bereich von 6 bis 8, wobei der pH-Wert vorzugsweise durch Zugabe einer starken Base bei 7,8 konstant gehalten wird. Das Substrat ist unter den Reaktionsbedingungen stabil. Der Verlauf und der Endpunkt der enzymatischen Reaktion kann durch die Neutralisation der entstehenden $H^+$ -Ionen bestimmt werden. Die Neutralisation kann durch anorganische Basen als auch durch organische Basen erfolgen.

Nach Beendigung der enzymatischen Reaktion werden die Produkte, der N-Acyl-D-ester oder das N-Acyl-D-amid und die N-Acyl-L-säure der Phenylglycine aus dem wäßrigen Reaktionsmedium durch Ausschütteln oder Ausrühren mit organischen Lösungsmitteln extrahiert. Die Trennung der D-Ester bzw. D-Amide von den L-Säuren erfolgt in üblicher Weise durch Alkalischstellen der Reaktionslösung und Extrahieren der Ester bzw. Amide mit einem organischen Lösungsmittel wie Chloroform, Essigsäureäthylester, Methylenchlorid oder Butylacetat. Die verbleibende wäßrige Phase wird dann sauer gestellt, beispielsweise mit Schwefelsäure, und erneut mit Essigester extrahiert. Die erhaltenen Verbindungen werden dann auf ihre optische Reinheit überprüft.

Zur Herstellung von D- bzw. L-Phenylglycin werden die beiden Verbindungen 4 Stunden bei 80°C in 2n Salzsäure erhitzt. Nach dem Abkühlen der Lösung wird diese mit Natronlauge oder wäßrigem Ammoniak auf pH 6 eingestellt und auf 4°C abgekühlt. Das auskristallisierte Phenylglycin wird abfiltriert, getrocknet und auf seine optische Reinheit untersucht.

Eis ist bereits bekannt geworden, daß mikrobielle und tierische Serinproteasen wie Carlsberg- und Novo-Subtilisine oder Chymotrypsin einige N-Acyl-L-aminosäureester spalten können (A. O. Barel und A. N Glazer, J. Biol. Chem., 243 (1968) 1344 bis 1348 sowie US—PS 3 963 573).

Diese unterscheiden sich jedoch von den erfindungsgemäß zu spaltenden Derivaten des Phenylglycins erheblich.

Nach Chemical Reviews *46* (1950), S. 69 bis 153, insbesondere 119 bis 122, waren ähnliche Ergebnisse bei acylierten Phenylglycinestern nicht zu erwarten.

Beispiel 1

Herstellung von N-Acetyl-DL-phenylglycinmethylester 41,4 g DL-Phenylglycinmethylesterhydrochlorid (0,205 Mol) werden in 200 ml Wasser gelöst. Die Lösung wird auf 4°C abgekühlt und mit einer gesättigten $NaHCO_3$-Lösung auf pH 6 bis 7 eingestellt. Es werden langsam 25 ml Essigsäureanhydrid (0.26 Mol) unter Rühren zugetropft und der pH-Wert durch gleichzeitige Zugabe der $NaHCO_3$-Lösung zwischen 6 und 7 gehalten. Es bildet sich ein weißer Niederschlag. Die Suspension wird mit Wasser auf 1,5 l aufgefüllt und mit 1 l Chloroform oder Methylenchlorid portionsweise ausgerührt. Die vereinigten Chlolformphasen werden über $Na_2SO_4$ getrockent, filtriert und mit Hilfe eines Rotationsverdampfers weitgehendst eingeengt. Der ausgefallene N-Acylester wird im Vakuum über $P_2O_5$ und Paraffin getrocknet. Man erhält 36,0 g (84,7% der Theorie) vom Schmelzpunkt 79 bis 80°C.

Die Dünnschichtchromatographie auf Keiselgelplatten im Lösungsmittelsystem n-Butanol/$CH_3$ $COOH/H_2O$ = 4/1/1 ergab eine mit Jod anfärbbare Zone mit dem $R_f$-Wert von 0,59 bis 0,61. Die hergestellte Verbindung reagierte nicht mit Ninhydrin. Das NMR-Spektrum entsprach der Formel.

Beispiel 2

Herstellung von N-Acetyl-DL-phenylglycinäthylester Abs 112,4 g DL-$\alpha$-Phenylglycinäthylester HCl (0,52 Mol) wurden wie im Beispiel 1 beschrieben 75,6 g N-Acetyl-DL-phenylglycinäthylester (67,3% der Theorie) vom Schmelzpunkt 63°C erhalten.

Die Verbindung reagiert nich mit Ninhydrin und ist im Dünnschichtchromatogramm nach Anfärbung mit Jod einheitlich ($R_f$-Wert = 0,61). Das NMR-Spektrum entspricht der Formel.

Beispiel 3

Herstellung von N-Acetyl-DL-4-hydroxyphenylglycinmethylester

25 g DL-4-Hydroxyphenylglycinmethylesterhydrochlorid (0,115 Mol) wurden wie im Beispiel 1 beschrieben zu 12,8 g N-Acetyl-DL-4-hydroxyphenylglycinmethylester (50,1% der Theorie) vom Schmelzpunkt 112 bis 115°C umgesetzt.

Mittels Dünnschichtchromatographie im Lösungsmittelsystem aus Beispiel 1 ist neben einer nu mit Jod anfärbbaren Zone ($r_f$-Wert = 0,59 bis 0,61) noch eine Spur einer weiteren Zone, die mit Ninhydrin und Jod reagiert, nachweisbar ($R_f$-Wert = 0,31). Diese Zone entspricht einem geringen Anteil an nicht umgesetzten DL-4-Hydroxyphenyglycinmethylester.

Beispiel 4

Herstellung von Subtilisin-Anhydridharz

30 g mit Aceton gewaschenes Anhydridharz (Zusammensetzung: 80 Gew.-% Tetraäthylenglykoldimethacrylat, 10 Gew.-% Methacrylsäure und 10 Gew.-% Maleinsäureanhydrid) werden in 400 ml Wasser suspendiert und mit 40 ml wäßriger Triäthylaminlösung versetzt. Der pH-Wert wird mit 0,1 M Essigsäure auf 6,2

eingestellt und unter Rühren wird 1 g Subtilisin (Alcalase Novo, 6,59 Anson-Einheiten/g, Hersteller: Novo AS, Kopenhagen/Dänemark zugefügt. Der pH-Wert wird durch Titration mit 1N NaOH auf 6,2 gehalten. Nach 12 Stunden wird das Enzymharz abgesaugt und auf der Nutsche mehrmals mit 0,05 M Phosphatpuffer + 1NaCl pH 7,5 und 0,5 M Phosphatpuffer pH 7,5 gewaschen. Von der ursprünglich eingesetzten Aktivität von 53.000 ATEE-Einheiten im Filtrat (40,4%) nachweisbar und 6,466 ATEE-Einheiten (12,2%) am Enzymharz kovalent gebunden.

Beispiel 5

Herstellung von Subtilisin-Anhydridharz (quervernetzt mit Glutardialdehyd)

9 g feuchtes Anhydridharz aus Beispiel 4 werden in 90 ml Wasser suspendiert und auf pH 5,7 eingestellt. Es werden unter Rühren 1,35 g Subtilisin (Alcalase Novo, 6,59 Anson-Einheiten/g, Hersteller: Novo AS, Kopenhagen/Dänemark) zugegeben. Das Harz wird 12 Stunden mit 0,1 N NaOH auf pH 5,7 gehalten und anschließend abgesaugt. Unter Rühren werden 50 ml 0,625% GlutardialdehydLösung zugegeben, das Harz nach 3 Stunden Reaktion bei Raumtemparatur abgesaugt und mit 0,1 M Phosphatlösung pH 7,0 gewaschen. Von 71.550 ATEE-Einheiten des löslichen Subtilisins sind 6.250 ATEE/g an das Enzymharz (8,7%) gebunden.

Beispiel 6

10 g feuchtes Amberlite XAD—7 Harz wird in 50 ml Wasser mit 20 mM $CaCl_2$ suspendiert und unter Rühren 1 g Maxatase (Maxatase, 500 000 Delft-Einheiten/g, Hersteller; Gist-Brocades N.V., Niederlande) zugefügt. Nach 2 Stunden Rühren bei Raumtemperatur wird abgesaugt und die adsorbierte Enzymaktivität bestimmt. Es werden 43,1% der eingesetzten Aktivität an das Harz adsorbiert. Das Enzymharz wird anschließend 2 Stunden mit einer 1,5 %igen Glutardialdehyd-Lösung quervernetzt und darauf portionsweise mit ingesamt 500 ml $H_2O$ + 20 mM $CaCl_2$ gewaschen. Es werden 9,6 g feuchtes quervernetztes Enzymharz erhalten, das 36,8% der ursprünglich eingesetzten Aktivität gebunden enthielt.

Beispiel 7

Herstellung von Chymotrypsin-Anhydridharz

10 g Anhydridharz werden nach Beispiel 4 mit 1 g lyophilisiertem $\alpha$-Chymotrypsin vom Rind (Merck, Darmstadt/Bundesrepublik Deutschland) zur Reaktion gebracht. Das Enzym hat eine Aktivität von 105.800 ATEE-Einheiten/g. Im Filtrat der Enzymkupplung können 87 210 ATEE-Einheiten (82,4% der eingesetzten Aktivität nachgewiesen werden. Am Anhydridharz verbleiben 5 427 ATEE-Einheiten/g Enzymharz (5,1% der eingesetzten Aktivität).

Beispiel 8

Enzymatische Spaltung von N-Acetyl-DL-phenylglycinmethylester mit Subtilisin-Anhydridharz sowie anschließender saurer Hydrolyse zu D- und L-Phenylglycin

15 g nach Beispiel 1 hergestellter N-Acetyl-DL-Phenylglycinmethylester (72,38 m Mol) werden in 150 ml Dioxan-Wassergemisch (25 Vol.-% Dioxan) gelöst und 15 g nach Beispiel 4 hergestelltes Subtilisin-Anhydridharz unter Rühren zugegeben. Der pH-Wert wird durch Zugabe von 1 N NaOH mit einer Methrom-Titrationseinheit (pH-Meter E 300 B, Impulsomat E 473 und Dosimat 412, Metrohm, Herisau/Schweiz) bei pH 7,8 konstant gehalten. Nach einer Reaktionszeit von ca. 19 Stunden bei 25°C sind 38,0 ml 1 N NaOH verbraucht, was einer enzymatischen Spaltung von 52,5% des eingesetzten N-Acetyl-DL-phenyl-glycinmethylesters entspricht.

Das Subtilisin-Harz wird über eine Fritte abgesaugt und bis zur weiteren Verwendung bei 4°C gelagert. Das Filtrat wird mit festem $Na_2CO_3$ auf pH 9 eingestellt, dreimal mit 200 ml Essigester extrahiert, die vereinigten Extrakte mit $Na_2SO_4$ getrocknet. Der Essigester wird am Rotationsverdampfer abgezogen und die ausgefallene Substanz im Vakuum über $P_2O_5$ getrocknet. Man erhält, 6,08 g (81,0% der Theorie) vom Schmelzpunkt 107°C mit einem optischen Drehwert

$$[\alpha]^{25°C}_{578\ nm} = -174{,}7°$$

(c = 1 in Äthanol)

Die Dünnschichtchromatographie auf Kieselgelplatten im Lösungsmittelsystem n-Butanol/$CH_3COOH$/$H_2O$ = 4/1/1 ergibt eine mit Jod, jedoch nich mit Ninhydrin anfärbbare Zone, die der des Ausgangsmaterials entspricht ($R_f$-Werte = 0,61).

Die wäßrige Phase der ersten Extraktion wird mit 6N $H_2SO_4$ auf pH 2 eingestellt, dreimal mit 200 ml Essigester extrahiert, die vereinigten Extrakte mit $Na_2SO_4$ getrocknet und der Essigester am Rotationsverdampfer abgezogen. Die ausgefallene Substanz wird im Vakuum über $P_2O_5$ getrocknet. Man erhält 6,81 g (97,4% der Theorie) vom Schmelzpunkt 198°C mit einem Drehwert von

$$[\alpha]^{25°C}_{578\ nm} = +154{,}9°$$

(c = 1 in Äthanol)

Die Dünnschichtchromatographie unter den gleichen Bedingungen wie vorher gibt eine mit Jod anfärbbare Zone, die der von synthetischem N-Acetyl-Phenylglycin entspricht ($R_f$-Wert: 0,48).

Je 3 g N-Acetyl-D-phenylglycinmethylester und N-Acetyl-L-phenylglycin werden in 30 ml 2 N HCl gelöst und 4 Stunden bei 80°C erhitzt. Nach dem Abkühlen der Lösung wird der pH-Wert mit 10 N NaOH auf 6 eingestellt, das ausgefallene Phenylglycin abfiltriert und über $P_2O_5$ im Vakuum getrocknet.

Die Ausbeute an Phenylglycin aus N-Acetyl-D-phenylglycinmethylester beträgt 1,99 g (91,3% der Theorie).

Im Dünnschichtchromatogramm ist nur eine mit Jod und Ninhydrin anfärbbare Zone nachweisbar. Der $R_f$-Wert von 0,28 bis 0,29 gleicht dem vom synthetischen Phenylglycin. Der Drehwert beträgt

$$[\alpha]^{25°C}_{578\ nm} = -152,3°$$

(c = 0,6 in 2N HCl)

Die Ausbeute an Phenylglycin aus N-Acetyl-L-phenylglycin ist 2,26 g (96,2% der Theorie) mit dem $R_f$-Wert = 0,28 bei nur einer mit Jod und Ninhydrin anfärbbaren Zone.

$$[\alpha]^{25°C}_{578\ nm} = +104,3°$$

(c = 0,6 in 2N HCl)

Beispiel 9

Enzymatische Spaltung von N-Acetyl-DL-phenylglycinmethylester mit Chymotrypsin-Anhydridharz sowie anschließender saurer Hydrolyse zu D- und L-Phenylglycin

15 g nach Beispiel 1 hergestellter N-Acetyl-DL-phenylglycinmethylester (72,38 m Mol) werden wie in Beispiel 8 beschrieben mit 15 g Chymotrypsin-Anhydridharz, hergestellt nach Beispiel 6, gespalten. Nach 17 Stunden und 20 Minuten Reaktionszeit bei 25°C werden 37,5 ml 1N NaOH verbraucht, was einer enzymatischen Spaltung von 51,8% des eingesetzten N-Acetyl-DL-phenylglycinmethylesters entspricht.

Das Enzymharz und die Lösungen werden wie in Beispiel 7 beschrieben aufgearbeitet.

Ausbeute an N-Acetyl-D-phenylglycinmethylester vom Schmelzpunkt 102 bis 106°C: 7,46 g (99,5% der Theorie).

$$[\alpha]^{25°C}_{578\ nm} = -156,5°$$

(c = 1 in Äthanol)

Im Dünnschichtchromatogramm ist nur eine mit Jod anfärbbare Zone mit dem $R_f$-Wert von 0,61 nachweisbar.

Ausbeute an N-Acetyl-L-phenylglycin vom Schmelzpunkt 193 bis 196°C: 6,33 g (90,6% der Theorie).

$$[\alpha]^{25°C}_{578\ nm} = +188,5°$$

(c = 1 in Äthanol)

Im Dünnschichtchromatogramm ist nur eine mit Jod anfärbbare Zone mit dem $R_f$-Wert 0,48 bis 0,49 nachweisbar.

Die saure Hydrolyse von je 4 g N-Acetyl-DL-phenylglycinmethylester und N-Acetyl-L-phenylglycin zu D- bzw. L- Phenylglycin erfolgt wie in Beispiel 8 angegeben.

Ausbeute an D-Phenylglycin: 2,52 g (86,3% der Theorie).

$$[\alpha]^{25°C}_{578\ nm} = -132°$$

(c = 0,6 in 2N HCl)

Die im Dünnschichtchromatogramm mit Ninhydrin und Jod anfärbbare Zone entspricht mit einem $R_f$-Wert von 0,28 derjenigenvon synthetischem Phenylglycin.

Ausbeute an L-Phenylglycin: 3,03 g (96,8% der Theorie).

$$[\alpha]^{25°C}_{578\ nm} = +134°$$

(c = 0,6 in 2N HCl)

Im Dünnschichtchromatogramm ist nur eine mit Jod und Ninhydrin anfärbbare Zone und der $R_f$-Wert 0,28 nachweisbar.

Beispiel 10

Enzymatische Spaltung von N-Acetyl-DL-phenylglycinäthyl-ester mit Subtilisin-Anhydridharz sowie anschließender saurer Hydrolyse zu D- und L-Phenylglycin

10 g nach Beispiel 2 hergestellter N-Acetyl-DL-phenylglycinäthylester (45,19 m Mol) werden wie in Beispiel 8 in 100 ml Dioxan-Wasser (3:7, volumetrisch) gelöst und mit 15 g Subtilisin-Anhydridharz (hergestellt nach Beispiel 5) versetzt. Es wird wie in Beispiel 8 mit 1N NaOH auf pH 7,8 titriert. Nach 16 Stunden Reaktionszeit bei 25°C sind 20,8 ml 1N NaOH verbraucht, was einer enzymatischen Spaltung von 46,0 % des eingesetzten N-Acetyl-DLphenylglycinäthylesters entspricht.

Die weitere Aufarbeitung des Ansatzes wurde wie in Beispiel 8 beschrieben vorgenommen.

Ausbeute an N-Acetyl-Phenylglycin-äthylester vom Schmelzpunkt 86°C: 4, 83 g (96,6% der Theorie).

$$[\alpha]^{25°C}_{578\,nm} = -151{,}9°$$

(c = 1,56% in Äthanol)

Im Dünnschichtchromatogramm wurde eine nur mit Jod anfärbbare Zone mit dem $R_f$-Wert von 0,59 gefunden, die dem Ausgangsmaterial entspricht.

Ausbeute an N-Acetyl-L-phenylglycin vom Schmelzpunkt 191°C: 4,04 g (92,4 % der Theorie).

$$[\alpha]^{25°C}_{578\,nm} = +194{,}7°$$

(c = 1 in Äthanol)

Im Dünnschichtchromatogramm war nur eine mit Jod anfärbbare Zone mit dem $R_f$-Wert von 0,49 nachweisbar, was auch dem $R_f$-Wert von synthetischem N-Acetyl-phenylglycin entspricht.

Die enzymatische Spaltung wurde 5 mal mit demselben Enzymharz jeweils mit neuem Substrat wiederholt, wobei immer das gleiche Ergebnis erhalten wurde.

Die saure Hydrolyse von je 4 g N-Acetyl-D-phenylglycinäthylester und N-Acetyl-L-phenylglycin zu D- bzw. L-phenylglycin erfolgte wie in Beispiel 8 angegeben.

Ausbeute an D-Phenylglycin: 1,96 (71,8% der Theorie).

$$[\alpha]^{25°C}_{578\,nm} = -164{,}5°$$

(c = 0,6 in 2N HCl)

Ausbeute an L-Phenylglycin: 2,07 g (75,8% der Theorie).

$$[\alpha]^{25°C}_{578\,nm} = +140{,}8°$$

(c = 0,6 in 2N HCl)

$R_f$-Werte = 0,28.

Beispiel 11

Enzymatische Spaltung von N-Acetyl-DL-4-hydroxy-phenylglycinmethylester mit Subtilisin-Anhydridharz sowie anschließender saurer Hydrolyse zu D- und L-p-hydroxy-phenylglycin

5 g nach Beispiel 3 hergestellter N-Acetyl-DL-4-hydroxy-phenylglycinmethylester (22,4 m Mol) werden in 70 ml Dioxan/Wasser (3:7, volumetrisch) gelöst und wie in Beispiel 8 beschrieben mit 10 g Subtilisin-Anhydridharz gespalten. Nach 6,5 Stunden Reaktionszeit bei 25°C sind 12,3 ml 1N NaOH verbraucht, was einer enzymatischen Spaltung von 54,9% des Ausgangsmaterials enspricht.

Das Enzymharz und die Lösungen werden wie in Beispiel 8 beschrieben aufgearbeitet.

Ausbeute an N-Acetyl-D-4-hydroxy-phenylglycinmethylester vom Schmelzpunkt 168 bis 170°C: 2,14 g (85,6% der Theorie).

$$[\alpha]^{25°C}_{578\,nm} = -163{,}2°$$

(c = 1 in Äthanol)

Im Dünnschichtchromatogramm ist nur eine mit Jod anfärbbare Zone mit dem $R_f$-Wert 0,62 nachweisbar.

Ausbeute an N-Acetyl-L-4-hydroxy-phenylglycin vom Schmelzpunkt 194 bis 197°C 1,81 g (77,2 % der Theorie).

$$[\alpha]^{25°C}_{578\,nm} = +181{,}0°$$

(c = l in Methanol)

Im Dünnschichtchromatogramm war eine nur mit Jod anfärbbare Zone mit dem $R_f$-Wert 0,50 nachweisbar.

Je 1,5 g N-Acetyl-D-4-hydroxy-phenylglycinmethylester und N-Acetyl-L-4-hydroxy-phenylglycin wurden wie in Beispiel 7 beschrieben sauer hydrolysiert.

Ausbeute an 4-Hydroxy-phenylglycin aus N-Acetyl - D - 4 - hydroxy - phenylglycin: 0,74 g (66,1% der Theorie).

Im Dünnschichtchromatogramm ist nur eine mit Jod und Ninhydrin anfärbbare Zone mit dem $R_f$-Wert 0,25 nachweisbar, was dem von synthetischen 4-Hydroxy-phenylglycin entspricht.

Ausbeute an 4-Hydroxy-phenylglycin aus N-Acetyl - L - 4 - hydroxy - phenylglycin: 0,76g (63,3% der Theorie).

Im Dünnschichtchromatogramm ist nur eine mit Jod und Ninhydrin anfärbbare Zone mit dem $R_f$-Wert von 0,25 bis 0,27 nachweisbar.

**Patentansprüche**

1. Verfahren zur stereoselektiven Spaltung von DL-Phenylglycinestern durch Hydrolyse der Estergruppe an N-Acyl-L-phenylglycinestern in N-Acyl-DL-phenylglycinestern durch Einwirkung von Enzymen, Trennung der N-Acyl-D-phenylglycinester von den N-Acyl-L-phenylglycinen und gegebenenfalls anschließende saure Hydrolyse der Estergruppen der D-Enantiomeren sowie der Acylgruppen, dadurch gekennzeichnet, daß man auf die N-Acyl-DL-phenylglycinester in Lösung trägergebundene proteolytische Enzyme einwirken läßt.

2. Verfahren nach Anspruch 1, dadurch gekennzeichnet, daß die zu spaltenden Verbindungen der Formel

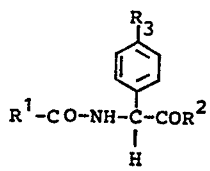

DL-Form

entsprechen, worin

$R^1$ Wasserstoff, den Rest einer gegebenenfalls substituierten aliphatischen oder araliphatischen Monooder Dicarbonsäure oder einer natürlichen oder synthetischen $\alpha$-Aminocarbonsäure,

$R^2$ Alkoxy und

$R^3$ Wasserstoff, Hydroxy, Alkoxy, Aralkoxy, Aryloxy, Cycloalkoxy oder Acyloxy bedeuten.

3. Verfahren nach Anspruch 1, dadurch gekennzeichnet, daß die zu spaltende Verbindung der Formel

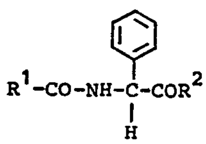

DL-Form

entspricht, worin

$R^1$ Wasserstoff, Mono-, Di- und Trichlormethyl, Trifluormethyl oder $\omega$-Carboxy-$C_2$-$C_6$-alkyl und

$R_2$ Methoxy oder Äthyoxy bedeuten.

4. Verfahren nach Anspruch 1, dadurch gekennzeichnet, daß die zu spaltende Verbindung der Formel

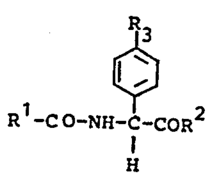

DL-Form

entspricht, worin

$R^1$ Wasserstoff Methyl, Mono-, Di- und Trichlormethyl, Trifluormethyl oder $\omega$-Carboxy-$C_2$-$C_6$-alkyl,

$R^2$ Methoxy oder Athyoxy und

$R^3$ Hydroxy, Methoxy, Äthoxy oder Acetoxy bedeuten.

5. Verfahren nach Anspruch 1, dadurch gekennzeichnet, daß als Enzyme Serin- und Sulfhydryl-Proteasen verwendet werden.

6. Verfahren nach Anspruch 1, dadurch gekennzeichnet, daß als Enzyme Subtilisin (EC 3.4.4.16), $\alpha$-Chymotrypsin (EC 3.4.4.5), Papain (EC 3.4.4.10), Ficin oder Bromelain verwendet werden.

7 Verfahren nach Anspruch 1, dadurch gekennzeichnet, daß als Enzym Subtilisin verwendet wird.

8. Verfahren nach Anspruch 1, dadurch gekennzeichnet, daß die Spaltung bei einer Temperatur von 20—40°C und in einem pH-Bereich von 6—8 durchgeführt wird.

9. Verfahren nach Anspruch 1, dadurch gekennzeichnet, daß man als Träger für die trägergebundenen Enzyme Copolymerisate aus Methacrylaten, Methacrylsäure und Maleinsäureanhydrid, gegegbenenfalls durch Glutardialdehyd quervernetzt, verwendet.

10. Verfahren nach Anspruch 6, dadurch gekennzeichnet, daß man als Träger für die trägergebundenen Enzyme, insbesondere Subtilisin, ein Copolymerisat aus Tetraäthylenglykoldimethacrylat, Methacrylsäure und Maleinsäureanhydrid, gegebenenfalls mit Glutardialdehyd quervernetzt, verwendet.

## Revendications

1. Procédé de scission stéréosélective d'esters de DL-phénylglycine par hydrolyse du groupe ester porté par des esters de N-acyl-L-phénylglycine en esters de N-acyl-DL-phénylglycine par l'action d'enzymes, séparation des esters de N-acyl-D-phénylglycine des N-acyl-L-phénylglycines et, le cas échéant, hydrolyse acide subséquente des groupes ester des D-énantiomères ainsi que des groupes acyle, caractérisé en ce qu'on fait agir des enzymes protéolytiques liés à un support sur les esters de N-acyl-DL-phénylglycine en solution.

2. Procédé suivant la revendication 1, caractérisé en ce que les composés à scinder répondent à la formule:

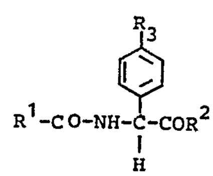

forme DL

dans laquelle

$R^1$ désigne un atome d'hydrogène, le reste d'un acide monocarboxylique ou dicarboxylique aliphatique ou araliphatique éventuellement substitué ou d'un acide $\alpha$-aminocarboxylique naturel ou synthétique,

$R^2$ est un groupe alkoxy et

$R^3$ est un atome d'hydrogène ou un groupe

hydroxy, alkoxy, aralkoxy, aryloxy, cycloalkoxy ou acyloxy.

3. Procédé suivant la revendication 1, caractérise en ce que le composé à scinder répond à la formule:

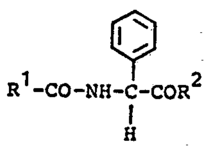

forme DL

dans laquelle

$R^1$ est un atome d'hydrogène ou un groupe mono-, diou trichlorométhyle, trifluorométhyle ou $\omega$-carboxy-(alkyle en $C_2$ à $C_6$) et

$R_2$ est un groupe méthoxy ou éthoxy.

4. Procédé suivant la revendication 1, caractérisé en ce que le composé à scinder répond à la formule:

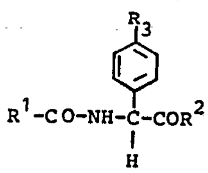

forme DL

dans laquelle

$R^1$ est un atome d'hydrogène ou un groupe méthyle, mono-, di- ou trichlorométhyle, trifluorométhyle ou $\omega$-carboxy-(alkyle en $C_2$ à $C_6$),

$R^2$ est un groupe méthoxy ou éthoxy, et

$R^3$ est un groupe hydroxy, méthoxy, éthoxy ou acétoxy.

5. Procédé suivant la revendication 1, caractérisé en ce qu'on utilise comme enzymes des sérine-protéases et des sulfhydryle-protéases.

6. Procédé suivant la revendication 1, caractérisé en ce qu'on utilise comme enzymes la subtilisine (EC 3.4.4.16), l'$\alpha$-chymotrypsine (EC 3.4.4.5), la papaïne (EC 3.4.4.10), la ficine ou la broméline.

7.Procédé suivant la revendication 1, caractérisé en ce qu'on utilise comme enzyme la subtilisine.

8. Procédé suivant la revendication 1, caractérisé en ce que la scission est conduite à une température de 20 à 40°C dans une plage de pH de 6 à 8.

9. Procédé suivant la revendication 1, caractérisé en ce qu'on utilise comme support pour les enzymes liés à ces supports, des copolymérisats de méthacrylates, d'acide méthacrylique et d'anhydride d'acide maléique, éventuellement réticulés par le dialdéhyde glutarique.

10. Procédé suivant la revendication 6, caractérisé en ce qu'on utilise comme support pour les enzymes liés à de tels supports, en particulier la subtilisine, un copolymérisat de diméthacrylate de tétraéthylèneglycol, d'acide méthacrylique et d'anhydride d'acide maléique, éventuellement reticulé avec le dialdéhyde glutarique.

**Claims**

1. Process for the stereoselective resolution of DL-phenylglycine esters by hydrolyzing the ester group on N-acyl-L-phenylglycine esters in N-acyl-DL-phenylglycine esters by the action of enzymes, separating the N-acyl-D-phenylglycine esters from the N-acyl-L-phenylglycines and then, if desired, hydrolyzing, under acid conditions, the ester groups of the D-enantiomers and the acyl groups, characterised in that proteolytic enzymes bonded to carriers are allowed to act on the N-acyl-DL-phenylglycine esters in solution.

2. Process according to claim 1, characterised in that the compounds to be resolved correspond to the formula

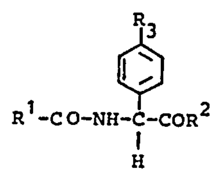

DL-Form

in which

$R^1$ denotes hydrogen, the radical of an optionally substituted aliphatic or araliphatic monocarboxylic or dicarboxylic acid or of a naturally occurring or synthetic $\alpha$-amino-carboxylic acid.

$R^2$ denotes alkoxy and

$R^3$ denotes hydrogen, hydroxy, alkoxy, aralkoxy, aryloxy, cyloalkoxy or acyloxy.

3. Process according to claim 1, characterised in that the compund to be resolved corresponds to the formula

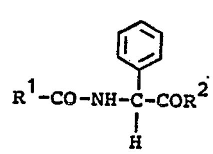

DL Form

in which

$R^1$ denotes hydrogen, mono-, di- or trichloromethyl, trifluormethyl or $\omega$-carboxy-$C_2$-$C_6$-alkyl and

$R^2$ denotes methoxy or ethoxy.

4. Process according to claim 1, characterised in that the compound to be resolved corresponds to the formula

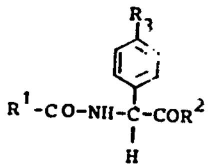

DL-Form

in which

$R^1$ denotes hydrogen, methyl, mono-, di- or trichloromethyl, trifluoromethyl or $\omega$-carboxy-$C_2$-$C_6$-alkyl,

$R^2$ denotes methoxy or ethoxy and

$R^3$ denotes hydroxy, methoxy, ethoxy or acetoxy.

5. Process according to claim 1, characterised in that serine proteases or sulphydryl proteases are used as the enzymes.

6. Process according to claim 1 characterised in that subtilisin (EC 3.4.4 16), $\alpha$-chymotrypsin (EC 3.4.4.5), papain (EC 3 4.4.10), ficin or bromelain are used as the enzymes.

7. Process according to claim 1. characterised in that subtilisin is used as the enzyme.

8. Process according to claim 1. characterised in that the resolution is carried out at a temperature of 20 to 40°C and in a pH range of 6 to 8.

9. Process according to claim 1, characterised in that the carriers used for the enzymes bonded to carriers are copolymers of methacrylates, methacrylic acid and maleic anhydride, optionally crosslinked by glutardialdehyde.

10. Process according to claim 6, characterised in that the carrier used for the enzymes bonded to carriers, in particular subtilisin, is a copolymer of tetraethylene glycol dimethacrylate, methacrylic acid and maleic anhydride, optionally crosslinked by glutardialdehyde.